Europäisches Patentamt

(19) European Patent Office

Office européen des brevets

(11) EP 0 796 612 B1

# (12) FASCICULE DE BREVET EUROPEEN

(45) Date de publication et mention
de la délivrance du brevet:
**17.03.1999 Bulletin 1999/11**

(51) Int. Cl.[6]: **A61K 7/42**, A61K 7/48

(21) Numéro de dépôt: **97400411.1**

(22) Date de dépôt: **25.02.1997**

(54) **Compositions cosmétiques comprenant des nanopigments**

Nanopigmente-enthaltende kosmetische Zusammensetzungen

Cosmetic compositions comprising nanopigments

(84) Etats contractants désignés:
**DE ES FR GB IT**

(30) Priorité: **20.03.1996 FR 9603457**
**17.04.1996 FR 9604803**

(43) Date de publication de la demande:
**24.09.1997 Bulletin 1997/39**

(73) Titulaire: **L'OREAL**
**75008 Paris (FR)**

(72) Inventeurs:
 • **Marchi-Lemann, Patricia**
  **75008 Paris (FR)**
 • **Collette, Annick**
  **94600 Choisy Le Roi (FR)**
 • **Mellul, Myriam**
  **94240 L'Hay-Les-Roses (FR)**

(74) Mandataire: **Dodin, Catherine**
**L'Oreal-D.P.I.,**
**90, rue du Général Roguet**
**92583 Clichy Cédex (FR)**

(56) Documents cités:
**EP-A- 0 503 922**   **EP-A- 0 523 294**
**EP-A- 0 755 670**   **WO-A-95/09895**

• **DATABASE WPI Section Ch, Week 8652 Derwent Publications Ltd., London, GB; Class A96, AN 86-343344 XP002018996 & JP 61 257 907 A (POLA KASEI KOGYO KK) , 15 Novembre 1986**
• **DATABASE WPI Section Ch, Week 9013 Derwent Publications Ltd., London, GB; Class A96, AN 90-096553 XP002018997 & JP 02 049 717 A (TORAY IND INC) , 20 Février 1990**

**Description**

L'invention a pour objet de nouvelles compositions cosmétiques comprenant des nanoparticules de pigments, des charges et un liant gras, ces compositions ayant des propriétés optiques, d'application et de protection anti-UV améliorées.

On connaît par les documents JP-06199636, JP-072615, EP-523294 des compositions cosmétiques : dispersions en milieu huile ou eau, émulsions eau dans huile (E/H) ou huile dans eau (H/E), comprenant des nanoparticules de pigment, essentiellement des nanoparticules de dioxyde de titane, et éventuellement des charges.

Le choix d'utiliser des nanoparticules de dioxyde de titane (TiO$_2$) est dicté par le souci d'obtenir une bonne protection contre les rayonnements ultra-violet (UV), sans être gêné par le blanchiment caractéristique du dioxyde de titane de taille ordinaire. Il est par ailleurs connu que les compositions à base de nanoparticules de pigments sont plus hétérogènes que les compositions à base de pigments de taille ordinaire, en raison d'une moins bonne dispersion des pigments ; elles sont par conséquent plus difficiles à étaler sur la peau. Les travaux décrits dans l'art antérieur ont cherché à résoudre ce problème.

Les compositions connues de l'art antérieur ne sont pas dépourvues d'inconvénients : Un léger blanchiment peut être observé lorsque la quantité de nanoparticules est élevée, en particulier dans le cas des produits à forte protection anti-UV. Ce défaut est particulièrement sensible dans les produits de soin dont les qualités esthétiques doivent être parfaites. Certaines compositions remédient à ce problème en associant un filtre anti-UV organique à des nanoparticules de TiO$_2$, ce qui proportionnellement permet d'utiliser moins de TiO$_2$. Toutefois l'utilisation topique quotidienne de compositions comprenant un ou plusieurs filtres anti-UV organiques peut être source d'allergies et peut provoquer une sensibilisation de la peau. En outre, contrairement aux filtres anti-UV minéraux, les filtres anti-UV organiques ne sont pas parfaitement stables dans le temps, ce qui induit une baisse de leur efficacité et un accroissement du risque de sensibilisation lié à ces produits.

Si l'on connaît déjà, en particulier par les documents JP-06199636, JP-072615 et EP-523294, des compositions à base de nanoparticules de pigments bien dispersées, en revanche on a pu observer dans ces compositions une réagglomération des nanoparticules de pigment au cours du temps, ce qui a pour conséquence une sensation rêche à l'étalement de la composition sur la peau et une dégradation au cours du temps des qualités esthétiques du produit.

La présente invention a pour but de proposer de nouvelles compositions cosmétiques à base de nanoparticules de pigments, parfaitement transparentes et exemptes de blanchiment lorsqu'on les applique sur la peau, douées de propriétés de filtre anti-UV suffisantes pour n'avoir pas recours à l'adjonction de filtres organiques, mieux dispersées que les compositions de l'art antérieur, cette qualité étant maintenue au cours du temps par la stabilité remarquable de ces compositions.

L'invention concerne plus particulièrement les compositions cosmétiques comprenant une quantité de nanopigments supérieure à 2 %, cette quantité étant mesurée en poids par rapport au poids total de la composition. En effet, en dessous d'une telle proportion de nanopigments, ceux-ci ne posent pas de problèmes de dispersion ni de stabilité dans les compositions les comprenant. En outre, des quantités trop faibles de nanopigments ne permettent pas d'obtenir une protection UV satisfaisante sans l'adjonction d'autres filtres anti-UV.

L'invention a pour objet de nouvelles compositions cosmétiques comprenant au moins :

- 2% en poids de pigment sous forme de nanoparticules que l'on désignera nanopigment,
- une charge,
- un liant gras,
  caractérisées en ce que :
- la concentration pigmentaire volumique (CPV) de l'ensemble constitué des nanopigments et des charges est inférieure ou égale à la concentration pigmentaire volumique critique (CPVC) tel que $CPV \leq CPVC/2$,
- le volume des charges Vc est supérieur ou égal à 2 fois le volume des nanopigments Vn.
  Il est connu de EP-A-755 670 des compositions comprenant des charges et des pigments et pour lesquelles

$$CPV \geq \frac{CPVC}{2}.$$

Par rapport aux compositions connues dans l'art antérieur, l'adjonction de charges aux nanopigments dans les conditions prescrites ci-dessus a pour effet d'améliorer la dispersion des nanopigments, aussi bien au moment de la préparation de la composition qu'au cours de son étalement ; la stabilité de cette dispersion est également améliorée. En outre, les qualités optiques des nanopigments sont améliorées par la contribution des propriétés optiques intrinsèques des charges. Enfin, les compositions selon l'invention, lorsqu'elles sont utilisées dans le maquillage, présentent des qualités esthétiques améliorée qui se traduisent par une plus grande homogénéité de la couleur, une coloration à la fois transparente et plus intense, et cela sans dégradation des qualités de ce produit dans le temps.

La composition selon l'invention comprend donc au moins une charge, au moins 2 % de nanopigment et un liant gras.

La CPV du mélange charges et nanopigments doit être inférieure ou égale à la CPVC.

Si l'on désigne par V le volume de l'ensemble constitué des charges et des nanopigments, et V' le volume de la fraction non volatile du liant gras, la concentration pigmentaire volumique ou CPV de l'ensemble constitué des nanopigments et des charges est définie par :

$$CPV = \frac{V}{V+V'} \times 100$$

On rappelle que la prise d'huile est mesurée en déterminant le volume Vh de la fraction non volatile du liant gras juste nécessaire pour combler les interstices entre les particules de la poudre (charge plus nanopigment).La prise d'huile peut être mesurée par exemple selon la norme américaine ASTM D281-84. La concentration pigmentaire volumique critique (CPVC) est alors définie par :

$$CPVC = \frac{V}{V+Vh} \times 100$$

De façon préférentielle les compositions selon l'invention vérifient la relation :

$$CPV \leq CPVC/2,$$

et encore plus préférentiellement :

$$CPV \leq CPVC/3$$

De façon avantageuse on choisit des compositions qui vérifient :

$$CPV = CPVC/3 \pm 10\%$$

Dans la composition selon l'invention, on choisit également $Vc \geq 2Vn$ .

L'ensemble de ces critères de sélection permet d'obtenir des compositions dont les propriétés de protection UV, de transparence, de coloration, de facilité d'étalement sont très améliorées par rapport à l'art antérieur.

Les particules de nanopigments utilisées dans la présente invention sont de forme indifférente. Par nanopigments, on entend des pigments dont la taille moyenne des particules élémentaires est comprise entre 0,01 à 0,15 microns lorsque ces particules sont sensiblement sphériques, préférentiellement elles ont une taille moyenne allant de 0,01 à 0,06 microns et de façon avantageuse de 0,01 à 0,03 microns. Lorsque les particules de nanopigments ne sont pas sphériques, elles ont préférentiellement une plus grande dimension inférieure à 0,15 microns et avantageusement comprise entre 0,02 et 0,10 microns. De tels nanopigments d'oxyde de titane correspondent à des nanopigments d'oxyde de titane connus, habituellement utilisés dans le domaine cosmétique comme filtres.

Les nanopigments utilisés dans la présente invention peuvent être choisis parmi les nanoparticules de pigments minéraux et de pigments organiques, comme par exemple parmi les nanoparticules d'oxyde de fer, de dioxyde de titane, d'oxyde de zinc, d'oxychlorure de bismuth, de silicate de calcium, d'oxyde de chrome, d'hydroxyde de chrome, de ferrocyanure d'ammonium ferrique, de ferrocyanure ferrique, de kaolin, de violet de manganèse, d'ultramarine, de noir de carbone et leurs mélanges. De préférence on choisit des nanoparticules d'oxydes métalliques. Avantageusement, ces oxydes métalliques sont choisis parmi les oxydes de titane, de zinc et/ou de fer, qui font barrière aux UV.

Les charges utilisables dans la présente invention sont des charges minérales ou organiques de préférence de grande taille, préférentiellement de taille supérieure ou égale à 10 microns. On peut citer comme exemples de charges minérales le talc, la silice, le mica, le nitrure de bore, et comme exemples de charges organiques la poudre de Nylon, la poudre de silicone et la poudre de polyméthylène méthacrylate. Préférentiellement on choisira des charges lamellaires.

Le liant gras est choisi de manière usuelle parmi les huiles et les cires d'origine animale, végétale ou synthétique et leurs mélanges. Dans le cas où le liant est solide à température ambiante, comme par exemple dans le cas des cires, la prise d'huile est mesurée à la température de fusion du liant.

On peut citer parmi les huiles utilisables dans la présente invention, l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide des huiles d'hydrocarbures telles que des huiles de paraffine, le squalane, la vaseline ; des esters tels que le myristate d'isopropyle, le palmitate d'isopropyle, le stéarate de butyle, le laurate d'hexyle, l'isononanoate d'isononyle, le palmitate de 2-éthyl hexyle, le laurate de 2-hexyl décyle, le palmitate de 2-octyl décyle, le myristate de 2-octyl dodécyle, le succinate de 2-diéthylhexyle, le malate de diisostéaryle, le lactate de 2-octyl dodécyle, le triisostéarate de glycérine, etc. ; des huiles de silicone comme les polyméthyl siloxanes, les polyméthylphénylsiloxanes, des polysiloxanes modifiés par des acides gras, des polysiloxanes modifiés par des alcools gras, des polysiloxanes modifiés par des polyoxyalkylènes, des silicones fluorées, etc.; des huiles perfluorées et/ou organofluorées ; des acides gras supérieurs tels que l'acide myristique, l'acide palmitique, l'acide stéarique, l'acide béhénique, l'acide oléique, l'acide linoléique, l'acide linolénique, l'acide isostéarique, des alcools gras supérieurs tels que l'alcool cétylique, l'alcool stéarylique, l'alcool oléique etc ; les cires peuvent être choisies notamment parmi la cire de Carnauba, la cire de Candelilla, la cire d'abeille, la cire de baleine, les cires microcristallines, les lanolines etc.

Le liant gras peut contenir en outre des huiles volatiles, qui s'évaporeront au contact de la peau, mais dont la présence dans la composition cosmétique est utile car elles facilitent l'étalement de la composition lors de l'application sur la peau. De tels agents d'étalement appelés ici huiles volatiles sont généralement des huiles ayant à 25°C une tension de vapeur saturante au moins égale à 0,5 millibar (soit $0,5.10^2$Pa).

Les huiles volatiles lorsqu'elles sont présentes représentent généralement moins de 10% en poids de la composition finale et moins de 20% en poids du liant gras.

Parmi les huiles volatiles utilisables dans la présente invention on citera par exemple des huiles de silicone telles que l'hexaméthyldisiloxane, le cyclopentadiméthylsiloxane, le cyclotétraméthylsiloxane, des huiles fluorées comme celles commercialisées sous la dénomination Galden (Montefluos) ou des huiles isoparaffiniques telles que celles qui sont commercialisées sous la dénomination ISOPAR (E, G, L ou H).

Les compositions cosmétiques selon l'invention peuvent se présenter sous forme de composition anhydre coulée, de dispersion huileuse, d'émulsion eau dans huile, d'émulsion huile dans eau, d'émulsion eau dans cire et d'émulsion cire dans eau. Les émulsions cire dans eau et eau dans cire ne sont réalisables selon la présente invention que dans la mesure où l'on peut mesurer à chaud la CPVC du mélange nanopigments et charges pour la cire ou le mélange de cires concernées, aussi on choisit de préférence des cires ou des mélanges de cires de faible cohésion cristalline.

De façon habituelle, les compositions selon l'invention comprennent de 2 à 30% en poids du mélange nanopigments + charges par rapport au reste des composants. Préférentiellement, l'ensemble nanopigments et charges représente de 5 à 15% en poids de la composition.

De façon préférentielle, lorsque la composition selon l'invention est une émulsion E/H ou H/E, afin d'optimiser la dispersion des nanopigments et des charges, la composition est préparée selon un procédé comprenant au moins deux étapes, la première étape consistant à préparer une dispersion, à partir d'un mélange homogène des nanopigments et des charges tels que définis ci-dessus, dans une partie du liant gras de la composition. La quantité du liant gras à utiliser dans cette première étape du procédé est évaluée par l'homme du métier en fonction de la quantité de nanoparticules et de charges, de façon à obtenir à l'issue de cette première étape une pâte suffisamment souple pour pouvoir être broyée à la tricylindre. Ce procédé est également objet de l'invention.

De façon préférentielle, les compositions selon l'invention comprennent en outre un agent dispersant, cet agent contribuant à une meilleure dispersion et une meilleure stabilité de la formule. Les agents dispersants utilisables dans la présente invention sont ceux répondant à la formule X-CO-AR, dans laquelle A représente un radical divalent, R est une amine primaire, secondaire ou tertiaire ou le sel d'une amine avec un acide ou un ammonium quaternaire et X représente un résidu polyester, le groupement X-CO- étant dérivé d'un acide hydroxycarboxylique de formule $HOR_1COOH$ dans lequel $R_1$ représente un groupement hydrocarbyle saturé ou insaturé. On peut citer en exemple de tels agents dispersants des dérivés de l'acide ricinoléique, de l'acide hydroxystéarique, l'acide gras de l'huile de castor hydrogénée. On peut également utiliser des agents dispersants à base d'un ou plusieurs résidus polyesters ou à base des sels d'un acide carboxylique ou d'un acide hydroxycarboxylique. Sont également utilisables les monoesters d'alcanolamines d'acides gras avec des acides carboxyliques. Par exemple les alcanolamines peuvent être choisies parmi l'éthanolamine, la propanolamine, ou l'aminoéthyl éthanolamine. On peut également choisir des agents dispersants parmi ceux qui sont à base de polymères ou de copolymères d'acide acrylique ou d'acide méthacrylique ainsi que ceux ayant un groupement époxy dans leur constituant de base comme ceux préparés à partir des esters de phosphate éthoxylés.

La quantité d'agent dispersant utilisé va généralement de 5 à 35% en poids par rapport au poids de charge et de nanopigment, préférentiellement de 5 à 20% en poids par rapport au poids de charge et de nanopigment.

Les compositions selon l'invention peuvent également comprendre les ingrédients habituellement utilisés en cosmétique parmi lesquels on peut citer les tensioactifs, qu'ils soient non-ioniques, cationiques, anioniques ou amphotères, des adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les matières colorantes. Les quantités de ces différents adjuvants sont celles classiquement utilisées dans les domaines considérés, et par exemple de 0,01 % à 20 % du poids total de la composition. Ces adjuvants, selon leur nature, peuvent être introduits dans la phase grasse, dans la phase aqueuse et/ou dans les sphérules lipidiques.

Comme exemple de tensioactifs utilisables dans l'invention on peut citer les émulsionnants à base de silicone et les émulsifiants lipidiques comme les alcools gras, les acides gras, les esters de glycérol, les esters de sorbitan, les esters de méthylglycoside et les esters de saccharose.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (Carbomer), les copolymères acryliques tels que les copolymères d'acrylates/alkyl acrylates, les polyacrylamides, les polysaccharides tels que l'hydroxypropyl cellulose, les gommes naturelles (xanthane) et les argiles, et, comme gélifiants lipophiles, on peut citer les argiles modifiées comme les bentones, les sels métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon, les extraits bactériens ou végétaux, notamment d'Aloe Vera.

Comme actifs lipophiles, on peut utiliser le tocophérol (vitamine E) et ses dérivés, les acides gras essentiels, les céramides, les huiles essentielles.

On peut, entre autre, introduire dans les compositions selon l'invention des agents actifs destinés notamment à la prévention et/ou au traitement des affections cutanées. Parmi ces agents actifs, on peut citer à titre d'exemple :

- les agents modulant la différenciation et/ou la prolifération et/ou la pigmentation cutanée tels que la vitamine D et ses dérivés, les oestrogènes tels que l'oestradiol, l'acide kojique ou l'hydroquinone ;
- les agents anti-radicaux libres, tels que l'alpha-tocophérol ou ses esters, les superoxyde dismutases, certains chélatants de métaux ou l'acide ascorbique et ses esters.

Ces compositions constituent notamment des crèmes teintées transparentes, des crèmes et des laits de protection solaire, des fonds de teint, des rouges à lèvres, des eye-liners, des mascaras, des produits de soin pour les lèvres, des poudres coulées.

Exemples :

Les pourcentages donnés dans les exemples sont des pourcentages en poids,

Exemples 1 à 16 :

Des émulsions huile dans eau sont préparées à partir de :

| A1 : | |
|---|---|
| - monostéarate de glycéryle/ stéarate de polypolyéthylène glycol (50/50) | 2,10 |
| - monostéarate de sorbitan oxyéthyléné | 0,90 |
| - alcool cétylique | 0,50 |
| - acide stéarique | 1,50 |
| - huile de vaseline | z |
| - para-hydroxybenzoate de propyle | 0,20 |
| A2 : | |
| - nanopigment de dioxyde de titane | 5,00 |
| - huile de vaseline | x |
| - charge | y |
| B : | |
| - eau | qsp 100 |
| - glycérine | 3,00 |
| - parahydroxybenzoate de propyle | 0,25 |
| C : | |
| - cyclopentadiméthylsiloxane | 3,00 |
| - xanthane | 0,20 |
| - polymère carboxyvinylique | 0,15 |

en faisant varier la nature de la charge, x, y et z.

Les différentes charges utilisées sont décrites dans le tableau I :

TABLEAU I

| Référence | Nom commercial | Producteur | Nature | Taille | Forme |
|---|---|---|---|---|---|
| C1 | Ceram Blanche 1 | SPCI | Nitrure de bore | 0,1 à 0,5 $\mu$m | Lamellaires |
| C2 | Orgasol 2002 UDNatCos | ATOCHEM | Nylon | 5 $\mu$m | Sphériques |
| C3 | Ceram Blanche | SPCI | Nitrure de bore | 3 à 6 $\mu$m | Lamellaires |
| C4 | Orgasol 2002 DNatCos | ATOCHEM | Nylon | 11 $\mu$m | Sphériques |
| C5 | Talc P3 | NIPPON TALC | Talc | 1,8 $\mu$m | Lamellaires |
| C6 | Tospearl 108 | TOSHIBA | Silicone | 0,8 $\mu$m | Sphériques |
| C7 | Mica SFG70 | ASPANGER | Mica | | Lamellaires |
| C8 | Talc Luzenac 00 | LUZENAC | Talc | 15 $\mu$m | Lamellaires |
| C9 | Microballoon Type H40 | MAPRECOS | Silice | 50 $\mu$m | Sphériques |
| C10 | Silica Beads SB150 | MAPRECOS | Silice | 3 - 15 $\mu$m | Sphériques |
| C11 | Tospearl 3120 | TOSHIBA | Silicone | 12 $\mu$m | Sphériques |
| C12 | Micropearl M-100 | SEPPIC | PMMA | 10 - 20 $\mu$m | Sphériques |

6

TABLEAU I (suite)

| Réfé-rence | Nom commercial | Producteur | Nature | Taille | Forme |
|---|---|---|---|---|---|
| C13 | Mica Concord 1000 | SCIAMA | Mica | 10 $\mu$m | Lamellaires |
| C14 | SG Flake 3M | MAPRECOS | Silice | 3 $\mu$m | Lamellaires |
| C15 | Covabead PMMA | WACKHERR | PMMA | < 5 $\mu$m | Sphériques |
| C16 | Chemicelen | SUMITOMO | Silice | 12 $\mu$m | Lamellaires |

Le pourcentage en volume de charge Vc est défini par rapport au pourcentage en volume de dioxyde de titane Vn.

On choisit l'une ou l'autre des valeurs Vc/Vn=R1=0,84  et  Vc/Vn=R2=2,81 .

A partir de ces pourcentages en volume et connaissant les densités des charges (environ 2 g/cm$^3$) et du dioxyde de titane (3,97 g/cm$^3$), on peut facilement calculer la valeur de y.

Le pourcentage en poids d'huile x+z est calculé de telle sorte que la composition vérifie CPV/CPVC=1/2 ou CPV/CPVC=1/3 .

Les pourcentages respectifs x et z des fractions d'huile introduites dans A1 et A2 sont adaptés à chaque cas pour que la phase A2 forme une pâte qui peut être facilement tricylindrée.

Dans un premier temps on prépare la pâte pigmentaire A2 en introduisant petit à petit x% d'huile de paraffine dans le mélange homogène charge+nanotitane. On tricylindre trois fois cette pâte. On chauffe le mélange des composants de A1 à 80°C et on introduit A2 dans ce mélange sous agitation puis on introduit le mélange A1+A2 dans B à 80°C et enfin on ajoute C à 40°C au reste de la composition. On laisse le mélange s'homogénéiser sous agitation et revenir à température ambiante. On obtient un lait de protection solaire dont les propriétés sont rapportées dans le tableau II.

Les références des charges C1 à C15 du tableau II se rapportent aux définitions données dans le tableau I.

On a analysé l'absorption et la transmission des rayonnements UV à différentes longueurs d'onde par mesure de la densité optique au spectrophotomètre Lambda 16 (Perkin Elmer) sur un échantillon de 6 mg placées entre deux lames de quartz, avec un étalement d'environ 2mg/cm$^2$.

L'aspect des compositions au microscope a été classé suivant 5 catégories numérotées de 1 à 5 :

1=mauvaise dispersion, 5=très bonne dispersion.

La viscosité est mesurée en poises.

La douceur, l'effet blanchissant et la rapidité de disparition du blanchiment observé à l'étalement ont été évalués sur un panel de 10 personnes, on donne une valeur moyenne de 1 à 5 :

-   douceur : 1=rêche, 5=très doux ;
-   blanchiment de la peau : 1=transparent, 5=blanc ;
-   disparition du blanchiment :1=très lent, 5=très rapide.

| n° ex | Charge | CPV ------- CPVC | Vc /Vn | ABSORPTION Visible 600 nm | UVA 350 nm | UVB 300 nm | TRANSMISSION Visible 600 nm | UVA 350 nm | UVB 300 nm | pH | Viscosité | Aspect | Douceur | Blanchi ment de la peau | Dispa rition du blanchi ment |
|---|---|---|---|---|---|---|---|---|---|---|---|---|---|---|---|
| 1 | C1 | 1/2 | R1 | 0,1 | 0,4 | 0,5 | 75,8 | 45,6 | 31,7 | 7,95 | 5,62 | 2 | 3,6 | 4,05 | 2,25 |
| 2 | C2 | 1/2 | R1 | 0,1 | 0,4 | 0,5 | 78,2 | 44,6 | 32,7 | 8 | 4,2 | 2 | 3,4 | 3,4 | 3,1 |
| 3 | C4 | 1/3 | R2 | 0,3 | 1,3 | 1,6 | 50,8 | 5,8 | 2,6 | 8,09 | 14 | 3 | 3,3 | 3,05 | 3,45 |
| 4 | C5 | 1/2 | R1 | 0,2 | 0,6 | 0,8 | 67,3 | 26,6 | 15,3 | 7,99 | 6 | 2 | 3,6 | 3,3 | 3,45 |
| 5 | C6 | 1/2 | R2 | 0,3 | 1,0 | 1,3 | 53,1 | 8,2 | 4,1 | 8,07 | 7,8 | 3 | 3,5 | 3,05 | 3,45 |
| 6 | C7 | 1/2 | R1 | 0,3 | 1,0 | 1,3 | 51,9 | 9,4 | 5,1 | 8 | 8,2 | 4 | 3,8 | 2,85 | 3,8 |
| 7 | C8 | 1/3 | R2 | 0,3 | 1,3 | 2,1 | 51,9 | 4,3 | 0,8 | 7,93 | 22,4 | 3 | 4,2 | 2,55 | 3,75 |
| 8 | C9 | 1/2 | R1 | 0,1 | 0,7 | 1,0 | 73,0 | 22,4 | 6,9 | 8,42 | 2,5 | 3 | 3,6 | 4,05 | 2,25 |
| 9 | C10 | 1/3 | R2 | 0,3 | 1,4 | 1,8 | 47,3 | 4,4 | 1,9 | 7,85 | 18,6 | 4 | 3,9 | 2,7 | 3,8 |
| 10 | C11 | 1/3 | R1 | 0,3 | 1,3 | 1,6 | 45,1 | 5,1 | 2,4 | 8,05 | 4,18 | 2 | 3,85 | 2,7 | 3,8 |
| 11 | C12 | 1/2 | R1 | 0,3 | 1,1 | 1,4 | 56,4 | 8,9 | 4,3 | 8,05 | 4,5 | 3 | 3,6 | 3,3 | 3,45 |
| 12 | C13 | 1/3 | R2 | 0,3 | 1,9 | 2,8 | 45,6 | 1,2 | 0,1 | 7,9 | 70 | 4 | 3,5 | 3,05 | 3,45 |
| 13 | C14 | 1/3 | R1 | 0,3 | 1,4 | 1,6 | 50,1 | 5,1 | 3,1 | 7,8 | 41,5 | 5 | 3,8 | 2,85 | 3,8 |
| 14 | C15 | 1/3 | R2 | 0,3 | 1,4 | 1,7 | 47,8 | 4,7 | 2,4 | 8,03 | 13,2 | 3 | 3,75 | 3,5 | 3,1 |
| 15 | C16 | 1/2 | R2 | 0,3 | 1,6 | 2,5 | 47,6 | 2,4 | 0,4 | 8,1 | 14,5 | 5 | 3,55 | 3,05 | 3,6 |

TABLEAU II

**Revendications**

1. Composition cosmétique comprenant une charge, un liant gras, et au moins 2% d'au moins un nanopigment, en poids par rapport au poids total de la composition, caractérisée en ce que :

   - la concentration pigmentaire volumique (CPV) de l'ensemble constitué des nanopigments et des charges est inférieure ou égale à la concentration pigmentaire volumique critique (CPVC) tel que $CPV \leq CPVC/2$,
   - le volume des charges Vc est supérieur ou égal à deux fois le volume des nanopigments Vn.

2. Composition selon la revendication 1, caractérisée en ce que les particules de nanopigment sont sensiblement sphériques et ont une taille moyenne allant de 0,01 à 0,15 microns.

3. Composition selon la revendication 1, caractérisée en ce que les particules de nanopigment ne sont pas sphériques et ont une plus grande dimension inférieure à 0,15 microns.

4. Composition selon l'une quelconque des revendications 1 à 3, caractérisée en ce que les nanopigments sont choisis parmi les oxydes métalliques.

5. Composition selon l'une quelconque des revendications 1 à 4, caractérisée en ce que les charges sont de taille supérieure ou égale à 10 microns.

6. Composition selon l'une quelconque des revendications 1 à 5, caractérisée en ce que les charges sont choisies parmi le talc, la silice, le mica, le nitrure de bore, la poudre de Nylon, la poudre de silicone et la poudre de polyméthylène méthacrylate.

7. Composition selon l'une quelconque des revendications 1 à 6, caractérisée en ce que la composition comprend de 2 à 30% en poids du mélange nanopigments + charges.

8. Composition selon l'une quelconque des revendications 1 à 7, caractérisée en ce qu'elle se présente sous la forme d'une crème teintée transparente, d'une crème et d'un lait de protection solaire, d'un fond de teint, d'un rouge à lèvres, d'un produit de soin pour les lèvres, d'un mascara, d'un eye-liner, d'une poudre coulée.

9. Utilisation cosmétique d'une composition selon l'une quelconque des revendications 1 à 8, pour la préparation d'un maquillage de couleur homogène, un maquillage ayant une coloration intense et/ou un maquillage transparent.

**Claims**

1. Cosmetic composition comprising a filler, a fatty binder and at least 2% by weight, with respect to the total weight of the composition, of at least one nanopigment, characterized in that:

   - the concentration of pigment by volume (CPV) of the combined nanopigments and fillers is less than or equal to the critical concentration of pigment by volume (CCPV), such that $CPV \leq CCPV/2$,
   - the volume of the fillers Vf is greater than or equal to twice the volume of the nanopigments Vn.

2. Composition according to Claim 1, characterized in that the nanopigment particles are substantially spherical and have a mean size ranging from 0.01 to 0.15 microns.

3. Composition according to Claim 1, characterized in that the nanopigment particles are not spherical and have a longer side of less than 0.15 microns.

4. Composition according to any one of Claims 1 to 3, characterized in that the nanopigments are chosen from metal oxides.

5. Composition according to any one of Claims 1 to 4, characterized in that the fillers have a size greater than or equal to 10 microns.

6. Composition according to any one of Claims 1 to 5, characterized in that the fillers are chosen from talc, silica, mica, boron nitride, nylon powder, silicone powder and poly(methyl methacrylate) powder.

7. Composition according to any one of Claims 1 to 6, characterized in that the composition comprises from 2 to 30% by weight of the nanopigments + fillers mixture.

8. Composition according to any one of Claims 1 to 7, characterized in that it is provided in the form of a transparent tinted cream, of a cream or of a milk for antisun protection, of a foundation, of a lipstick, of a product for caring for the lips, of a mascara, of an eyeliner or of a cast powder.

9. Cosmetic use of a composition according to any one of Claims 1 to 8 for the preparation of a makeup of homogeneous colour, a makeup having an intense colouring and/or a transparent makeup.

**Patentansprüche**

1. Kosmetische Zusammensetzung, die einen Füllstoff, ein Fettbindemittel und mindestens 2 Gew.-% mindestens eines Nanopigments, bezogen auf das Gesamtgewicht der Zusammensetzung, enthält, dadurch gekennzeichnet, daß:

   - die Volumenpigmentkonzentration VPK der gesamten Nanopigmente und Füllstoffe höchstens gleich der kritischen Volumenpigmentkonzentration KVPK ist, wie beispielsweise $VPK \leq KVPK/2$,
   - das Volumen der Füllstoffe $V_F$ mindestens das zweifache Volumen der Nanopigmente $V_N$ beträgt.

2. Zusammensetzung nach Anspruch 1, dadurch gekennzeichnet, daß die Nanopigmentpartikel im wesentlichen kugelförmig sind und eine mittlere Größe im Bereich von 0,01 bis 0,15 $\mu$m aufweisen.

3. Zusammensetzung nach Anspruch 1, dadurch gekennzeichnet, daß die Nanopigmentpartikel nicht kugelförmig sind und eine größere Anmessung unter 0,15 $\mu$m aufweisen.

4. Zusammensetzung nach einem der Ansprüche 1 bis 3, dadurch gekennzeichnet, daß die Nanopigmente unter den Metalloxiden ausgewählt sind.

5. Zusammensetzung nach einem der Ansprüche 1 bis 4, dadurch gekennzeichnet, daß die Füllstoffe eine Größe von mindestens 10 $\mu$m aufweisen.

6. Zusammensetzung nach einem der Ansprüche 1 bis 5, dadurch gekennzeichnet, daß die Füllstoffe unter Talk, Siliciumdioxid, Glimmer, Bornitrid, Nylonpulver, Siliconpulver und Polymethylenmethacrylatpulver ausgewählt sind.

7. Zusammensetzung nach einem der Ansprüche 1 bis 6, dadurch gekennzeichnet, daß die Zusammensetzung 2 bis 30 Gew.-% des Gemisches von Nanopigmenten und Füllstoffen enthält.

8. Zusammensetzung nach einem der Ansprüche 1 bis 7, dadurch gekennzeichnet, daß sie in Form einer transparenten, getönten Creme, einer Creme und einer Milch zum Sonnenschutz, eines Make-ups, eines Lippenstifts, eines Produkts zur Pflege der Lippen, einer Mascara, eines Eyeliners und eines gegossenen Pulvers vorliegt.

9. Kosmetische Verwendung einer Zusammensetzung nach einem der Ansprüche 1 bis 8 zur Herstellung einer Schminke von homogener Farbe, einer Schminke mit intensiver Färbung und/oder einer transparenten Schminke.